# EUROPEAN PATENT SPECIFICATION

(11) **EP 3 449 948 B1**
(45) Date of publication and mention of the grant of the patent: **02.02.2022**
(21) Application number: 18185392.0
(22) Date of filing: 25.07.2018
(51) Int. Cl.: A61L 2/10, A61L 2/24

(54) **OZONE-DISRUPTING ULTRAVIOLET LIGHT SANITIZING SYSTEMS AND METHODS**
OZONZERSTÖRENDE ULTRAVIOLETTLICHTREINIGUNGSSYSTEME UND VERFAHREN
SYSTÈMES ET PROCÉDÉS D'ASSAINISSEMENT À LA LUMIÈRE ULTRAVIOLETTE PERTURBANT LA COUCHE D'OZONE

(30) Priority: 30.08.2017 US 201715690338
(43) Date of publication of application: 06.03.2019
(73) Proprietor: The Boeing Company, Chicago, IL 60606-1596 (US)
(72) Inventor: Tillotson, Brian J., Chicago, 60606-1596 (US)
(74) Representative: Witte, Weller & Partner Patentanwälte mbB

(56) References cited:
- CN-A- 103 349 424
- CN-A- 104 873 994
- JP-A- H04 300 556
- JP-A- 2000 315 419
- US-A1- 2016 029 686
- US-B2- 9 144 618

## Description

### FIELD OF THE DISCLOSURE

Embodiments of the present disclosure generally relate to systems and methods for sanitizing surfaces with ultraviolet light, such as within lavatories of commercial aircraft, and, more particularly, to ozone-disrupting ultraviolet light sanitizing systems and methods.

Commercial aircraft are used to transport passengers between various locations. A typical commercial aircraft includes one or more lavatories within an internal cabin.

Systems are currently being developed to disinfect or otherwise sanitize surfaces within aircraft lavatories that use ultraviolet (UV) light. For example, it has been found that far UV light efficiently disinfects exposed surfaces within a lavatory.

Interaction of UV light with air creates ozone. As the UV light passes through air, the interaction of the UV light with oxygen molecules generates ozone molecules.

Ozone is an irritant, both to individuals and structures. For example, certain individuals may be susceptible to breathing disorders from prolonged exposure to ozone. Further, ozone is a reactive gas that may degrade surfaces of various structures.

Accordingly, the amount of ozone within confined spaces is typically controlled. The Federal Aviation Administration (FAA) provides regulations and guidelines regarding the presence of ozone onboard an aircraft. For example, an FAA regulatory guideline limits the amount of ozone within an internal cabin of an aircraft to an average of 100 parts ozone per billion over an eight hour timeframe. Further, the FAA regulatory guideline also limits the amount of ozone within an internal cabin of an aircraft to 250 parts ozone per billion within a three hour peak timeframe.

Accordingly, aircraft operators seek to limit the amount of ozone within an aircraft. One known disinfecting method limits the amount of generated ozone by placing a steriliz ing UV light in close proximity to a surface that is to be sterilized. For example, the UV light may be within one to six inches from a surface that is to be sterilized. The close proximity of the UV light to the surface limits ozone production, as the ozone travels through a shorter distance of ambient air. However, various structures are not able to be within such a close proximity to a UV light. For example, a UV light may not be effectively positioned within a few inches of a toilet or floor within a lavatory.

CN 104 873 994 A, according to its abstract, states a disinfecting and heating device for low-to-moderate-dangerousness medical equipment and a using method of the disinfecting and heating device. The disinfecting and heating device comprises a box body, a heater, an ultraviolet lamp and a bracket, wherein the box body is divided into a heating cavity and a disinfecting cavity which are communicated through an air outlet channel and an air return channel; the ultraviolet lamp and the bracket are arranged in the disinfecting cavity; the heater is arranged in the heating cavity; the air outlet channel is provided with a fan; the ultraviolet lamp has an ozone function. The medical equipment to be sterilized is arranged in the sterilizing cavity and is capable of keeping a warm state while rapid disinfection is realized, thus improving the comfort level of an inspected patient.

US 2016/029686 A1, according to its abstract, states that disinfecting devices and related methods are provided that apply germicidal ultraviolet light to disinfect dispensing components of food and beverage dispensers. A disinfecting holster for a bar gun includes a support surface configured to interface with a bar gun to support the bar gun when stowed in the holster, a housing coupled with the support surface and surrounding a dispensing nozzle of the bar gun when the bar gun is stowed in the holster, and an ultraviolet light source configured to emit germicidal ultraviolet light onto the nozzle. The housing substantially contains the ultraviolet light within the housing during the application of the ultraviolet light to the dispensing nozzle. The ultraviolet light can be periodically applied to maintain the nozzle in a disinfected state.

US 9,144,618 B2, according to its abstract, states sanitizing seat surfaces in a location being related to seating, such as a transportation vehicle includes a passenger area of the location. There are multiple rows of seats. A sanitization device includes a mobile body configured to travel along the aisle of the passenger area. An arm extends from the sanitization device laterally from the mobile body across the seats, and a source of UV radiation-mounted on the sanitization device is directed across the seats exposing surfaces in the passenger area to UV radiation.

Document CN 103 349 424 A is titled self-control toothbrush disinfection quick-dry placing device. Document JP H04 300556 A is titled deodorizing device. Document JP 2000315419 (A) is titled portable light source device.

Document KR 2004 43 128 Y is titled "Sanitary seat of a toilet bowl" and states in its abstract: A sanitary toilet seat that can dry, sterilize, and the like in the toilet seat. It can create a sanitary and clean toilet environment by installing a fan or a sterilizing lamp in the toilet to dry the seat and at the same time to sterilize and remove odors. A sanitary toilet seat is provided that toilet users can use with confidence by freeing themselves from anxiety about infection.

### SUMMARY OF THE DISCLOSURE

A need exists for a system and method of limiting the amount of ozone within a confined space. A need exists for a system and method of disrupting ozone generation within a confined space.

The claimed invention is directed to a lavatory including a UV light sanitizing according to claim 1 and a method of sanitizing the surfaces of structures of the lavatory according to claim 7.

The UV light sanitizing system includes a UV light assembly including a UV light source that is configured to emit UV light onto the surface of the structure, and an airflow generator that is configured to generate airflow within a region of UV light emission between the UV light source and the surface of the structure. The airflow generated by the airflow generator disrupts formation of ozone.

In at least one embodiment, the UV light sanitizing system includes a housing. The UV light assembly is secured within the housing. The airflow generator may be secured to the housing. Optionally, the airflow generator may be remotely located from the housing. For example, the airflow generator may be configured to be secured proximate to a portion of the structure

According to the invention, a UV light control unit is operatively coupled to the UV light assembly and the airflow generator. The UV light control unit is configured to control operation of the UV light assembly and the airflow generator. The UV light control unit is configured to activate the UV light source during a sanitizing cycle. The UV light control unit is configured to activate the airflow generator before the UV light source is activated during the sanitizing cycle.

In at least one embodiment, the airflow generator includes a fan assembly. The fan assembly may include at least one fan operatively coupled to at least one actuator.

The airflow generator is configured to generate airflow along and/or across the region of UV light emission.

Certain embodiments of the present disclosure provide a UV light sanitizing method that is configured to sanitize a surface of a structure within the lavatory. The UV light sanitizing method includes emitting UV light onto the surface of the structure with a UV light source of a UV light assembly, using an airflow generator to generate airflow within a region of UV light emission between the UV light source and the surface of the structure, and disrupting formation of ozone with the airflow generated by the airflow generator.

Certain embodiments of the present disclosure provide a vehicle that includes an internal cabin. A lavatory is within the internal cabin. The lavatory includes a structure. A UV light sanitizing system is disposed within the lavatory and configured to sanitize a surface of the structure.

### BRIEF DESCRIPTION OF THE DRAWINGS

Figure 1 illustrates a schematic diagram of an ultraviolet (UV) light sanitizing system, according to an embodiment of the present disclosure.
Figure 2 illustrates a schematic diagram of a UV light sanitizing system for an enclosed space, according to an embodiment of the present disclosure.
Figure 3 illustrates a simplified view of a UV light sanitizing system, according to an embodiment of the present disclosure.
Figure 4 illustrates a simplified view of a UV light sanitizing system, according to an embodiment of the present disclosure.
Figure 5 illustrates a simplified view of a UV light sanitizing system, according to an embodiment of the present disclosure.
Figure 6 illustrates a simplified view of a UV light sanitizing system, according to an embodiment of the present disclosure.
Figure 7 illustrates a flow chart of a method of sanitizing a component with UV light, according to an embodiment of the present disclosure.
Figure 8 illustrates a perspective front view of an aircraft, according to an embodiment of the present disclosure.
Figure 9 illustrates a perspective internal view of a lavatory, according to an embodiment of the present disclosure.

### DETAILED DESCRIPTION OF THE DISCLOSURE

The foregoing summary, as well as the following detailed description of certain embodiments will be better understood when read in conjunction with the appended drawings. As used herein, an element or step recited in the singular and preceded by the word "a" or "an" should be understood as not necessarily excluding the plural of the elements or steps. Further, references to "one embodiment" are not intended to be interpreted as excluding the existence of additional embodiments that also incorporate the recited features. Moreover, unless explicitly stated to the contrary, embodiments "comprising" or "having" an element or a plurality of elements having a particular condition may include additional elements not having that condition.

Certain embodiments of the present disclosure provide an ultraviolet (UV) light sanitizing system that includes a light source, such as a far UV light source, and a fan assembly that is configured generate airflow proximate to the UV light source and/or a surface of a component that is configured to be sterilized with UV light emitted from the UV light source. The airflow generated by the fan assembly disperses (for example, sweeps away) air away from the light source during operation, thereby eliminating, minimizing, or otherwise reducing ozone.

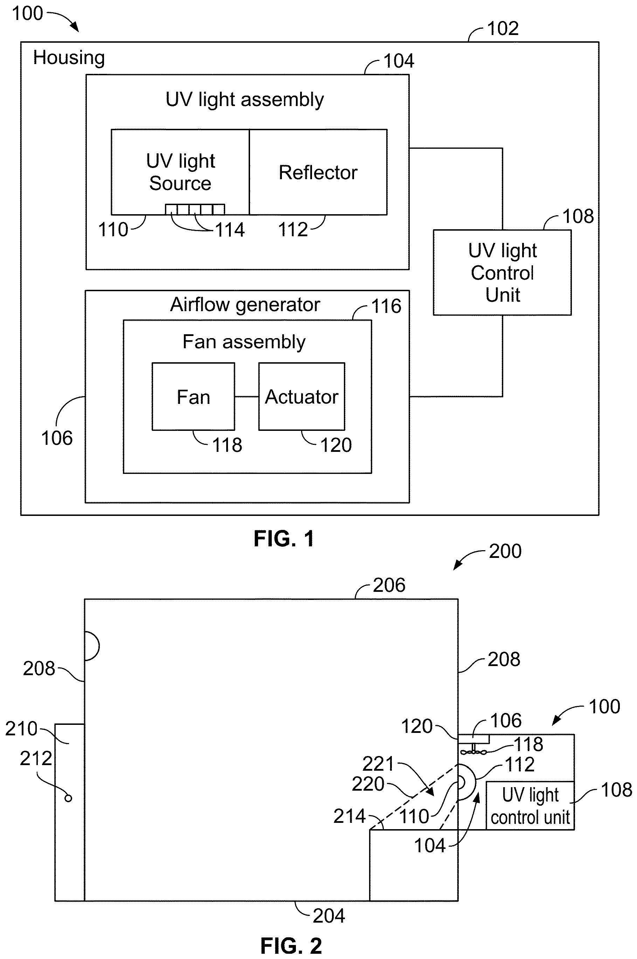

Figure 1 illustrates a schematic diagram of a UV light sanitizing system 100, according to an embodiment of the present disclosure. The UV light sanitizing system 100 includes a housing 102 that retains a UV light assembly 104, an airflow generator 106, and a UV light control unit 108. The UV light assembly 104, the airflow generator 106, and the UV light control unit 108 may be disposed within the housing 102, such as within an internal chamber defined by outer walls. Optionally, one or more of the UV light assembly 104, the airflow generator 106, and the UV light control unit 108 may be mounted on an outer surface of the housing 102. In at least one other embodiment, the airflow generator 106 and/or the UV light control unit 108 may be remotely located from the housing 102. For example, the airflow generator 106 may be secured on and/or proximate (for example, within less than 12.7 cm (five inches)) to a surface of a component that is configured to be sanitized by UV light emitted from the UV light assembly 104.

The UV light assembly 104 includes a UV light source 110 and a reflector 112. The UV light source 110 may be secured within a volume of space defined by the reflector 112. For example, the reflector 112 may be a parabolic reflector (such as formed of aluminum, and/or having internal mirror reflecting surfaces), and the UV light source 110 may be within an internal volume of space defined by the parabolic reflector. Optionally, the reflector 112 may be shaped differently than a parabola. As another example, the UV light source 110 itself may include the reflector 112. In at least one other embodiment, the UV light assembly 104 may not include the reflector 112.

The UV light source 110 is operatively coupled to the UV light control unit 108. The UV light source 110 may include one or more UV light elements 114, such as an arc lamp(s), laser(s), light emitting diode(s) (LEDs), microfilament(s), bulbs, fiber optic elements, and/or the like that are configured to emit UV light onto one or more structures within a confined space during a sanitizing cycle. In at least one embodiment, the UV light elements 114 are configured to emit far UV light. Alternatively, the UV light elements 114 may be configured to emit other types of UV light, such as UVA light, UVB light, UVC light, vacuum UV light, and/or the like. In at least one embodiment, the UV light source 110 may include UV light elements that are configured to emit UV light with different UV bands (for example, at different wavelengths and different frequencies). For example, one UV light element may be configured to emit far UV light, while another UV light element may be configured to emit UVC light.

The UV light control unit 108 is coupled to the UV light assembly 104 through one or more wired or wireless connections, and is configured to control operation of the UV light assembly 104. The UV light control unit 108 outputs an activation signal that is received by the UV light assembly 104, in particular the UV light source 110. The activation signal activates and controls the UV light source 110 during a sanitizing cycle in which the UV light source 110 emits UV light onto one or more structures within a confined space. The UV light control unit 108 may include or otherwise be coupled to a memory that stores data regarding the sanitizing cycle.

The airflow generator 106 is also operatively coupled to the UV light control unit 108, such as through one or more wired or wireless connections. In at least one embodiment, the airflow generator 106 includes a fan assembly 116 including a fan 118 (such as a rotor with blades) operatively coupled to an actuator 120 (such as an electric motor). The airflow generator 106 may be or include an electronic spooling fan, a bladeless fan, one or more oscillating vanes, and/or the like. The airflow generator 106 is configured to generate airflow in the region where UV light is emitted from the UV light source 110 and/or onto a surface where the emitted UV light is directed.

In operation, the UV light control unit 108 activates the UV light source 110 to emit UV light onto a surface to be sanitized during a sanitizing cycle. As the UV light source 110 is activated to emit UV light, the UV light control unit also activates the airflow generator 106 to generate airflow in the region of UV light emission (such as between the UV light source 110 and the surface to the sanitized by the emitted UV light). According to the claimed invention, when the UV light control unit 108 initiates the sanitizing cycle, the UV light control unit 108 first activates the airflow generator 106 to generate airflow before the UV light source 110 is activated to emit UV light. For example, the airflow generator 106 may be activated for one second before the UV light source 110 is activated, in order for the airflow generator 106 to achieve a full operational speed before UV light is emitted from the UV light source 110. Optionally, the airflow generator 106 may be activated for a period of less than one second before the UV light source 110 is activated.

The airflow generator 106 is operated during the sanitizing cycle. For example, the airflow generator 106 may be operated to generate airflow through and/or within a region of UV light emission for as long as the UV light source 110 emits UV light (such as for two or three seconds). In at least one embodiment, the airflow generator 106 may remain active to generate airflow for a predetermined period of time after the UV light source 110 is deactivated, such as for an additional one or two seconds.

As indicated, the airflow generator 106 may include the fan assembly 116, which may include the fan 118 operatively coupled to the actuator 120 The UV light control unit 108 may be operatively coupled to the actuator 120, such as through one or more wired or wireless connections. The actuator 120 may be an electronic or electric motor, one or more solenoids, or the like that causes the fan 118 to rotate, such as through a rotor operatively coupled to the actuator 120. Blades coupled to the rotor generate airflow as the rotor of the fan rotates. The fan 118 may cause airflow to be directed towards the UV light source 110, across the UV light source 110, and/or across an aperture or other such opening in the housing 102 through which UV light is emitted. For example, the fan 118 may operate to blow air onto and/or around the UV light source 110. Optionally, the fan 118 may operate to draw air into, onto and/or around the UV light source 110. The fan 118 may cause airflow to be directed towards a surface that is configured to be sanitized by the UV light emitted from the UV light source 110, and across the surface. For example, the fan 118 may operate to blow air onto and/or across the surface that is being sanitized by the UV light. Optionally, the fan 118 may operate to draw air into, onto, and/or around the surface that is being sanitized by the UV light.

In general, the airflow generator 106 is configured to generate airflow in at least a portion of a region of UV light emission between the UV light source 110 and the surface of a component that is being sanitized by the UV light emitted by the UV light source 110. The airflow generator 106 generates the airflow along and/or across the region of UV light generation.

The UV light sanitizing system 100 exploits a chemical process through which UV light converts oxygen (O₂) molecules into ozone (O₃) molecules. To create one ozone molecule, UV light photons excite two O₂ molecules from a ground state to an excited state. The two excited O₂ molecules typically bump into each other before they decay to a less-excited state. The rate of ozone production in a given volume is therefore not linear in relation to the concentration of excited O₂ molecules. Rather, the rate of ozone of production is proportional to the square of the concentration of excited O₂ molecules. In a typical lavatory application, UV light is concentrated in a small volume within the lavatory (for example, the region between the UV light source 110 and a surface, such as a countertop, that is to be disinfected). Almost all the excited O₂ molecules are produced in the small volume of space.

The airflow generator 106 disrupts production of ozone by generating airflow in at least a portion of the region of UV light generation, thereby dispersing the excited O₂ molecules. In doing so, the excited O₂ molecules are unlikely (or less likely) to bump into each other before they decay to a less-excited state. The airflow generator 106 generates the airflow proximate to (for example, within 15.24 or less cm (6 or less inches)) the UV light source 110 and/or the surface to be disinfected. The generated airflow then circulates throughout the enclosed space, carrying the excited O₂ molecules along with it, thereby dispersing the excited O₂ molecules from the region close to the UV light source 110 into an entire enclosed space, such as a lavatory.

For example, assume the volume ratio of the region of the UV light source 110 to an entire region of a lavatory is 1:100. Excited O₂ molecules with concentration X in the small region are diffused to a concentration of X/100 in the entire lavatory. Because the ozone production rate is proportional to the square of the concentration, the diffusion reduces the ozone production rate by a factor of 100² (that is, a factor of 10,000). The time constant for excited O₂ decaying to ground-state O₂ is unchanged, so decay becomes far more likely than ozone production. Such dramatic reduction in ozone production reduces the need for costly ozone countermeasures.

The UV light sanitizing system 100 provides efficient and effective sanitation through emission of UV light onto a surface. The UV light sanitizing system 100 saves energy, cycle time, and conditioned air, as compared to an alternative of completely flushing air from the enclosed space. Further, compared to using an ozone filter, the UV light sanitizing system 100 saves energy, cycle time, and maintenance time and effort.

As used herein, the term "control unit," "central processing unit," "CPU," "computer," or the like may include any processor-based or microprocessor-based system including systems using microcontrollers, reduced instruction set computers (RISC), application specific integrated circuits (ASICs), logic circuits, and any other circuit or processor including hardware, software, or a combination thereof capable of executing the functions described herein. Such are exemplary only, and are thus not intended to limit in any way the definition and/or meaning of such terms. For example, the UV light control unit 108 may be or include one or more processors.

The UV light control unit 108 is configured to execute a set of instructions that are stored in one or more data storage units or elements (such as one or more memories), in order to process data. For example, the UV light control unit 108 may include or be coupled to one or more memories. The data storage units may also store data or other information as desired or needed. The data storage units may be in the form of an information source or a physical memory element within a processing machine.

The set of instructions may include various commands that instruct the UV light control unit 108 as a processing machine to perform specific operations such as the methods and processes of the various embodiments of the subject matter described herein. The set of instructions may be in the form of a software program. The software may be in various forms such as system software or application software. Further, the software may be in the form of a collection of separate programs, a program subset within a larger program or a portion of a program. The software may also include modular programming in the form of object-oriented programming. The processing of input data by the processing machine may be in response to user commands, or in response to results of previous processing, or in response to a request made by another processing machine.

The diagrams of embodiments herein may illustrate one or more control or processing units, such as the UV light control unit 108. It is to be understood that the processing or control units may represent circuits, circuitry, or portions thereof that may be implemented as hardware with associated instructions (e.g., software stored on a tangible and non-transitory computer readable storage medium, such as a computer hard drive, ROM, RAM, or the like) that perform the operations described herein. The hardware may include state machine circuitry hardwired to perform the functions described herein. Optionally, the hardware may include electronic circuits that include and/or are connected to one or more logic-based devices, such as microprocessors, processors, controllers, or the like. Optionally, the UV light control unit 108 may represent processing circuitry such as one or more of a field programmable gate array (FPGA), application specific integrated circuit (ASIC), microprocessor(s), and/or the like. The circuits in various embodiments may be configured to execute one or more algorithms to perform functions described herein. The one or more algorithms may include aspects of embodiments disclosed herein, whether or not expressly identified in a flowchart or a method.

As used herein, the terms "software" and "firmware" are interchangeable, and include any computer program stored in a data storage unit (for example, one or more memories) for execution by a computer, including RAM memory, ROM memory, EPROM memory, EEPROM memory, and non-volatile RAM (NVRAM) memory. The above data storage unit types are exemplary only, and are thus not limiting as to the types of memory usable for storage of a computer program.

Figure 2 illustrates a schematic diagram of the UV light sanitizing system 100 for an enclosed space 200, according to an embodiment of the present disclosure. The enclosed space 200 may be defined by a floor 204, a ceiling 206, and walls 208 extending between the floor 204 and the ceiling 206. A door 210 may be moveably secured to one of the walls 208. The door 210 may include a lock 212 that is configured to securely lock the door 210 in a closed position. When the lock 212 is in a locked position, the door 210 is unable to be opened. When the lock 212 is in an unlocked position, the door 210 may be opened.

For example, the enclosed space 200 may be a lavatory onboard an aircraft. The enclosed space 200 may or may not include the door 210. The enclosed space 200 may be within various other vehicles, structures, and/or the like. For example, the enclosed space 200 may be a room within a commercial, municipal, or residential building, or a room onboard a train, bus, ship, or the like.

The enclosed space 200 includes at least one component 214 to be sanitized (for example, disinfected, sterilized, or otherwise cleaned) after use. For example, the component 214 may be a toilet, sink, floor, cabinet, wall, and/or the like within a lavatory of an aircraft.

As shown, the UV light sanitizing system 100 may be flush-mounted with one of the walls 208. Optionally, the UV light sanitizing system 100 may be mounted on an outer surface of the wall 208. In at least one other embodiment, the UV light sanitizing system 100 may be secured to the ceiling 206. In at least one other embodiment, the UV light sanitizing system 100 may be secured to the floor 204, and/or the component 214.

The UV light assembly 104 is configured to emit UV light 220 onto a surface of the component 214 during a sanitizing cycle. The UV light 220 is emitted in a region of UV light emission 221 between the UV light assembly 104 and the surface of the component 214 during the sanitizing cycle. The airflow generator 106, which may include the fan 118 operatively coupled to the actuator 120, generates airflow in at least a portion of the region of UV light emission (such as before, during, and/or after the emission of UV light from the UV light assembly 104), thereby disrupting generation of ozone, as described above.

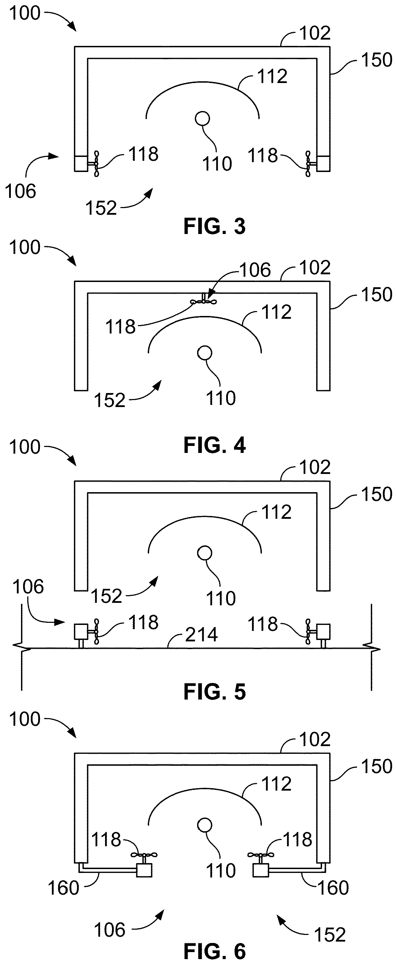

Figure 3 illustrates a simplified view of the UV light sanitizing system 100, according to an embodiment of the present disclosure. The housing 102 may include opaque outer walls 150 connected to a light outlet passage 152, such as an aperture or other such opening, a transparent window (such as formed of glass or clear plastic), and/or the like. The light source 110 may be secured to the housing 102 through fasteners, brackets, or other such mounting features. In at least one embodiment, the light source 110 may be secured to the reflector 112 through at least one retaining member, such as a socket(s), a bracket(s), a fastener(s), a guide track(s), rail(s), a clasp(s), a sleeve(s), and/or the like.

The airflow generator 106 may include one or more fans 118 secured to the housing 102 proximate to the light outlet passage 152. The fan(s) 118 may be oriented to blow and/or draw air across the light outlet passage 152. That is, the fan(s) 118 may be oriented and configured to blow and/or draw air across a portion of the region of UV light emission.

Figure 4 illustrates a simplified view of a UV light sanitizing system 100, according to an embodiment of the present disclosure. In this embodiment, the airflow generator 106 may be mounted to the housing 102 behind the UV light source 110. The airflow generator 106 may include one or more fans 118 that are configured to blow and/or draw air into and/or around the UV light source 110, thereby reducing ozone formation and cooling at the UV light source 110 at the same time.

Figure 5 illustrates a simplified view of a UV light sanitizing system 100, according to an embodiment of the present disclosure. In this embodiment, the airflow generator 106 may be remotely located from the housing 102. For example, the airflow generator 106 may be mounted onto and/or proximate to the component 214, and configured to blow and/or draw air onto and/or across the surface of the component 214 that is configured to be sanitized by the UV light emitted by the UV light source 110.

Figure 6 illustrates a simplified view of a UV light sanitizing system 100, according to an embodiment of the present disclosure. In this embodiment, the airflow generator 106 may be mounted to the housing 102 through one or more brackets 160. One or more fans 118 are directed toward the interior of the housing 102 and/or the UV light source 110.

Referring to Figures 1-6, before, and during, and/or after a sanitizing cycle, the airflow generator 106 generates airflow within at least a portion of a region of UV light emission between the UV light source 110 and the component 214 that is sterilized through the UV light generated by the UV light source 110. By generating the airflow within at least a portion of the region of UV light emission, excited oxygen molecules are dispersed throughout the enclosed space 200, thereby reducing the likelihood of ozone molecules forming.

The enclosed space 200 may also include vents and/or additional fans located therein and/or throughout. The vents and/or additional fans are configured to reduce air stagnation at areas within the enclosed space 200.

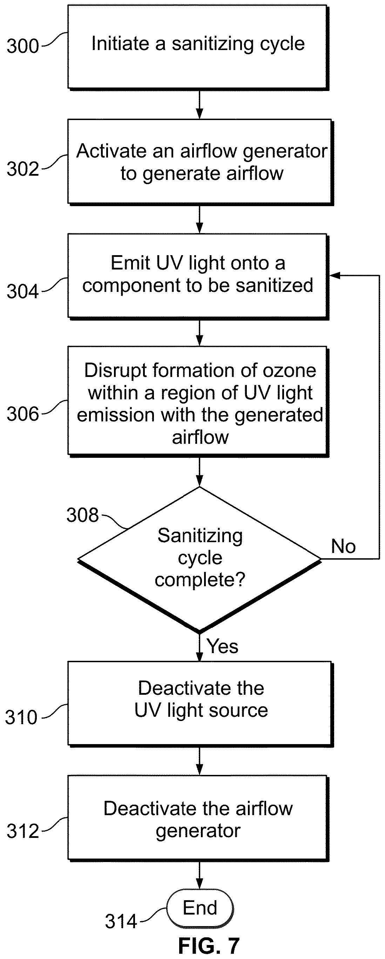

Figure 7 illustrates a flow chart of a method of sanitizing a component with UV light, according to an embodiment of the present disclosure. Referring to Figures 1 and 7, at 300, the UV light control unit 108 initiates a sanitizing cycle (such as after use of a lavatory onboard an aircraft by an individual). At 302, the UV light control unit 108 activates the airflow generator 106 to generate airflow. At 304, the UV light control unit 108 activates the UV light source 110 to emit UV light onto a component to be sanitized. Step 302 occurs prior to 304. At 306, formation of ozone is disrupted within a region of UV light emission by the generated airflow.

At 308, the UV light control unit 108 determines whether the sanitizing cycle is complete. If the sanitizing cycle is not complete, the method returns to 304. If, however, the sanitizing cycle is complete at 308, the method proceeds to 310, at which the UV light control unit 108 deactivates the UV light source 110. At 312, the UV light control unit 108 then deactivates the airflow generator 106. Step 310 may occur prior to step 312. Optionally, steps 310 and 312 may occur simultaneously. The method ends at 314.

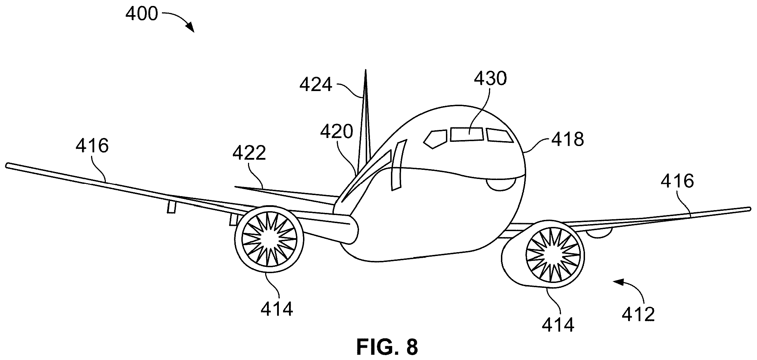

Figure 8 illustrates a perspective front view of an aircraft 400, according to an embodiment of the present disclosure. The aircraft 400 includes a propulsion system 412 that may include two turbofan engines 414, for example. Optionally, the propulsion system 412 may include more engines 414 than shown. The engines 414 are carried by wings 416 of the aircraft 400. In other embodiments, the engines 414 may be carried by a fuselage 418 and/or an empennage 420. The empennage 420 may also support horizontal stabilizers 422 and a vertical stabilizer 424.

The fuselage 418 of the aircraft 400 defines an internal cabin, which may include a cockpit 430, one or more work sections (for example, galleys, personnel carry-on baggage areas, and the like), one or more passenger sections (for example, first class, business class, and coach sections), and an aft section in which an aft rest area assembly may be positioned. Each of the sections may be separated by a cabin transition area, which may include one or more class divider assemblies. Overhead stowage bin assemblies may be positioned throughout the internal cabin. The internal cabin includes one or more chambers, such as lavatories, for example. One or more UV light sanitizing systems 100 (shown and described with respect to Figures 1-7, for example) may be located within the internal cabin.

Alternatively, instead of an aircraft, embodiments of the present disclosure may be used with various other vehicles, such as automobiles, buses, locomotives and train cars, watercraft, and the like. Further, embodiments of the present disclosure may be used with respect to fixed structures, such as commercial and residential buildings.

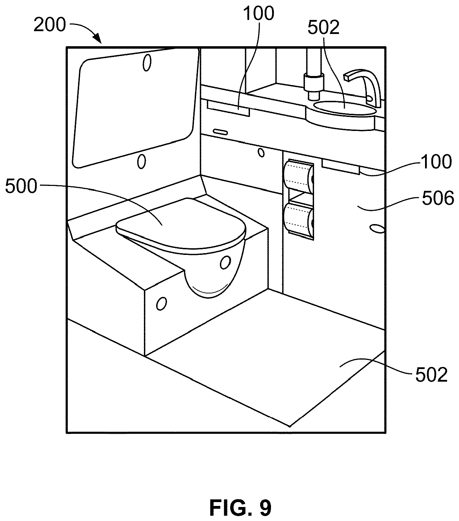

Figure 9 illustrates a perspective internal view of a lavatory 200, according to the claimed invention. As noted, the lavatory 200 is an example of the enclosed space 200 shown and described with respect to Figure 2, for example. The lavatory 200 may be onboard an aircraft, as described above. Optionally, the lavatory 200 may be onboard various other vehicles. In other embodiments, the lavatory 200 may be within a fixed structure, such as a commercial or residential building.

The lavatory 200 includes a base floor 502 that supports a toilet 500, cabinets 506, and a sink 502. UV light sanitizing systems 100 are secured within the lavatory 200 and are configured to be activated during a sanitizing cycle to sanitize (for example, disinfect, sterilize, or otherwise clean) various structures within the lavatory 200, such as the toilet 500, the floor 502, the cabinets 506, and/or the sink 502.

As described herein, embodiments of the present disclosure provide UV light sanitizing systems and methods that eliminate, minimize, or otherwise reduce ozone within a confined space. The UV light sanitizing systems and methods disrupt ozone generation within a confined space.

While various spatial and directional terms, such as top, bottom, lower, mid, lateral, horizontal, vertical, front and the like may be used to describe embodiments of the present disclosure, it is understood that such terms are merely used with respect to the orientations shown in the drawings. The orientations may be inverted, rotated, or otherwise changed, such that an upper portion is a lower portion, and vice versa, horizontal becomes vertical, and the like.

As used herein, a structure, limitation, or element that is "configured to" perform a task or operation is particularly structurally formed, constructed, or adapted in a manner corresponding to the task or operation. For purposes of clarity and the avoidance of doubt, an object that is merely capable of being modified to perform the task or operation is not "configured to" perform the task or operation as used herein.

It is to be understood that the above description is intended to be illustrative, and not restrictive. While the dimensions and types of materials described herein are intended to define the parameters of the various embodiments of the disclosure, the embodiments are by no means limiting and are exemplary embodiments. Many other embodiments will be apparent to those of skill in the art upon reviewing the above description. The scope of the various embodiments of the disclosure should, therefore, be determined with reference to the appended claims. In the appended claims, the terms "including" and "in which" are used as the plain-English equivalents of the respective terms "comprising" and "wherein." Moreover, the terms "first," "second," and "third," etc. are used merely as labels, and are not intended to impose numerical requirements on their objects.

This written description uses examples to disclose the various embodiments of the disclosure, including the best mode, and also to enable any person skilled in the art to practice the various embodiments of the disclosure, including making and using any devices or systems and performing any incorporated methods. The patentable scope of the various embodiments of the disclosure is defined by the claims.

## Claims

1. A lavatory (200) forming an enclosed space and including an ultraviolet (UV) light sanitizing system that is configured to sanitize a surface of a structure within the lavatory, such as a toilet (500), a floor (502), cabinets (506) and/or a sink (502), the UV light sanitizing system comprising:
a UV light assembly (104) including a UV light source (110) that is configured to emit UV light onto the surface of the structure, wherein the UV light by its UV light photons excites oxygen molecules from a ground state into an excited state; and
an airflow generator (106) that is configured to generate airflow proximate to the UV light source (110) and/or the surface to be sanitized and within a region of UV light emission between the UV light source (110) and the surface of the structure, wherein the generated airflow then circulates throughout the enclosed space, carrying the excited oxygen molecules along with it, thereby dispersing the excited oxygen molecules from the region close to the UV light source (110) into the entire enclosed space formed by the lavatory so that the airflow generated by the airflow generator (106) disrupts formation of ozone, wherein the UV light sanitizing system further comprises a UV light control unit (108) operatively coupled to the UV light assembly (104) and the airflow generator (106), wherein the UV light control unit (108) is configured to control operation of the UV light assembly (104) and the airflow generator (106), wherein the UV light control unit (108) is configured to activate the UV light source during a sanitizing cycle, wherein the UV light control unit (108) is configured to activate the airflow generator (106) before the UV light source is activated during the sanitizing cycle.

2. The lavatory of claim 1, the UV light sanitizing system further comprising a housing (102), wherein the UV light assembly (104) is secured within the housing (102).

3. The lavatory of claim 2, wherein the airflow generator (106) is secured to the housing (102).

4. The lavatory of any one of claims 1-3, wherein the airflow generator (106) comprises a fan assembly including at least one fan (118) operatively coupled to at least one actuator (120).

5. The lavatory of any one of claims 1-4, wherein the airflow generator (106) is configured to generate airflow along the region of UV light emission.

6. The lavatory of any one of claims 1-5, wherein the airflow generator (106) is configured to generate airflow across the region of UV light emission.

7. An ultraviolet (UV) light sanitizing method to sanitize a surface of a structure within a lavatory (200) such as a toilet (500), a floor (502), cabinets (506) and/or a sink (502), the UV light sanitizing method comprising:
emitting UV light onto the surface of the structure with a UV light source (110) of a UV light assembly (104) wherein the UV light converts oxygen molecules into ozone molecules by its UV light photons which excite oxygen molecules from a ground state into an excited state;
using an airflow generator (106) to generate airflow proximate to the UV light source (110) and/or the surface to be sanitized and within a region of UV light emission between the UV light source (110) and the surface of the structure, wherein the generated airflow then circulates throughout the enclosed space, carrying the excited oxygen molecules along with it, thereby dispersing the excited oxygen molecules from the region close to the UV light source (110) into the entire enclosed space formed by the lavatory so that the airflow generated by the airflow generator (106) disrupts formation of ozone;
wherein a UV light control unit (108) is operatively coupled to the UV light assembly (104) and the airflow generator (106);
controlling operation of the UV light assembly (104) and the airflow generator (106) by the UV light control unit (108);
activating the UV light source by the UV light control unit (108) during a sanitizing cycle; and
activating the airflow generator (106) by the UV light control unit (108) before the UV light source is activated during the sanitizing cycle.

8. The UV light sanitizing method of claim 7, further comprising securing the UV light assembly (104) within a housing (102).

9. The UV light sanitizing method of claim 7 or 8, further comprising securing the airflow generator (106) to a housing (102).

10. The UV light sanitizing method of any one of claims 7-9, further comprising remotely locating the airflow generator (106) from a housing (102), wherein the remotely locating comprises securing the airflow generator (106) within less than 12.7 cm (five inches) to a portion of the component.

11. The UV light sanitizing method of any one of claims 7-1 0, wherein the using the airflow generator comprises generating airflow along the region of UV light emission and/ the using the airflow generator comprises generating airflow across the region of UV light emission.

12. A vehicle comprising:
an internal cabin;
a lavatory of claim 1, wherein the airflow generator is configured to generate airflow one or both of along and across the region of UV light emission; and wherein the UV light sanitizing system further comprises:
a housing, wherein the UV light assembly is secured within the housing.

## Patentansprüche

1. Waschraumanordnung (200), die einen geschlossenen Raum bildet und ein Ultraviolett-Licht-(UV-Licht)-Desinfektionssystem aufweist, das dazu konfiguriert ist, eine Oberfläche einer Struktur innerhalb der Waschraumanordnung zu desinfizieren, wie einer Toilette (500), eines Bodens (502), von Schränken (506) und/oder eines Waschbeckens (502), wobei das UV-Licht-Desinfektionssystem aufweist:
eine UV-Licht-Anordnung (104), die eine UV-Licht-Quelle (110) aufweist, die dazu konfiguriert ist, UV-Licht auf die Oberfläche der Struktur zu emittieren, wobei das UV-Licht aufgrund seiner UV-Licht-Photonen Sauerstoffmoleküle von einem Grundzustand in einen angeregten Zustand anregt; und
einen Luftströmungs-Generator (106), der dazu konfiguriert ist, eine Luftströmung nahe der UV-Licht-Quelle (110) und/oder der zu desinfizierenden Oberfläche und innerhalb einer Region einer UV-Licht-Emission zwischen der UV-Licht-Quelle (110) und der Oberfläche der Struktur zu erzeugen, wobei die erzeugte Luftströmung dann durch den geschlossenen Raum zirkuliert, wobei sie die angeregten Sauerstoffmoleküle mit sich trägt, wodurch die angeregten Sauerstoffmoleküle aus der Region nahe der UV-Licht-Quelle (110) in dem gesamten geschlossenen Raum verteilt werden, der von der Waschraumanordnung gebildet wird, so dass die von dem Luftströmungs-Generator (106) erzeugte Luftströmung die Bildung von Ozon stört, wobei das UV-Licht-Desinfektionssystem ferner eine UV-Licht-Steuereinheit (108) aufweist, die betriebsmäßig mit der UV-Licht-Anordnung (104) und dem Luftströmungs-Generator (106) gekoppelt ist, wobei die UV-Licht-Steuereinheit (108) dazu konfiguriert ist, einen Betrieb der UV-Licht-Anordnung (104) und des Luftströmungs-Generators (106) zu steuern, wobei die UV-Licht-Steuereinheit (108) dazu konfiguriert ist, die UV-Licht-Quelle während eines Desinfektionszyklus zu aktivieren, wobei die UV-Licht-Steuereinheit (108) dazu konfiguriert ist, den Luftströmungs-Generator (106) während des Desinfektionszyklus zu aktivieren, bevor die UV-Licht-Quelle aktiviert wird.

2. Waschraumanordnung nach Anspruch 1, wobei das UV-Licht-Desinfektionssystem ferner ein Gehäuse (102) aufweist, wobei die UV-Licht-Anordnung (104) innerhalb des Gehäuses (102) festgelegt ist.

3. Waschraumanordnung nach Anspruch 2, wobei der Luftströmungs-Generator (106) an dem Gehäuse (102) festgelegt ist.

4. Waschraumanordnung nach einem beliebigen der Ansprüche 1 bis 3, wobei der Luftströmungs-Generator (106) eine Lüfteranordnung aufweist, die wenigstens einen Lüfter (118) beinhaltet, der betriebsmäßig mit wenigstens einem Aktuator (120) gekoppelt ist.

5. Waschraumanordnung nach einem beliebigen der Ansprüche 1 bis 4, wobei der Luftströmungs-Generator (106) dazu konfiguriert ist, eine Luftströmung entlang der Region einer UV-Licht-Emission zu erzeugen.

6. Waschraumanordnung nach einem beliebigen der Ansprüche 1 bis 5, wobei der Luftströmungs-Generator (106) dazu konfiguriert ist, eine Luftströmung quer über die Region einer UV-Licht-Emission zu erzeugen.

7. Ultraviolett-Licht-(UV-Licht)-Desinfektionsverfahren zum Desinfizieren einer Oberfläche einer Struktur innerhalb einer Waschraumanordnung (200), wie einer Toilette (500), einem Boden (502), von Schränken (506) und/oder einem Waschbecken (502), wobei das UV-Licht-Desinfektionsverfahren aufweist:
Emittieren von UV-Licht auf die Oberfläche der Struktur mittels einer UV-Licht-Quelle (110) einer UV-Licht-Anordnung (104), wobei das UV-Licht mittels seiner UV-Licht-Photonen, die Sauerstoffmoleküle von einem Grundzustand in einen angeregten Zustand anregen, Sauerstoffmoleküle in Ozon-Moleküle wandelt;
Verwenden eines Luftströmungs-Generators (106), um eine Luftströmung in der Nähe der UV-Licht-Quelle (110) und/oder der zu desinfizierenden Oberfläche und innerhalb einer Region von UV-Licht-Emission zwischen der UV-Licht-Quelle (110) und der Oberfläche der Struktur zu erzeugen, wobei die erzeugte Luftströmung dann durch den geschlossenen Raum zirkuliert, wobei sie die angeregten Sauerstoffmoleküle mit sich trägt, wodurch die angeregten Sauerstoffmoleküle aus der Region nahe der UV-Licht-Quelle (110) in dem gesamten geschlossenen Raum verteilt werden, der von der Waschraumanordnung gebildet wird, so dass die von dem Luftströmungs-Generator (106) erzeugte Luftströmung die Bildung von Ozon stört;
wobei eine UV-Licht-Steuereinheit (110) betriebsmäßig mit der UV-Licht-Anordnung (104) und dem Luftströmungs-Generator (106) gekoppelt ist;
Steuern eines Betriebs der UV-Licht-Anordnung (104) und des Luftströmungs-Generators (106) mittels der UV-Licht-Steuereinheit (108);
Aktivieren der UV-Licht-Quelle mittels der UV-Licht-Steuereinheit (108) während eines Desinfektionszyklus; und
Aktivieren des Luftströmungs-Generators (106) mittels der UV-Licht-Steuereinheit (108) während des Desinfektionszyklus, bevor die UV-Licht-Quelle aktiviert wird.

8. UV-Licht-Desinfektionsverfahren nach Anspruch 7, ferner mit dem Schritt des Festlegens der UV-Licht-Anordnung (104) innerhalb eines Gehäuses (102).

9. UV-Licht-Desinfektionsverfahren nach Anspruch 7 oder 8, ferner mit dem Schritt des Festlegens des Luftströmungs-Generators (106) an einem Gehäuse (102).

10. UV-Licht-Desinfektionsverfahren nach einem beliebigen der Ansprüche 7 bis 9, ferner mit dem Schritt, den Luftströmungs-Generator (106) entfernt von einem Gehäuse (102) anzuordnen, wobei das entfernte Anordnen beinhaltet, den Luftströmungs-Generator (106) innerhalb von weniger als 12,7 cm (fünf Zoll) bis hin zu einem Abschnitt der Komponente festzulegen.

11. UV-Licht-Desinfektionsverfahren nach einem beliebigen der Ansprüche 7 bis 10, wobei das Verwenden des Luftströmungs-Generators beinhaltet, die Luftströmung entlang der Region von UV-Licht-Emission zu erzeugen, und/oder wobei das Verwenden des Luftströmungs-Generators beinhaltet, eine Luftströmung quer über die Region der UV-Licht-Emission zu erzeugen.

12. Fahrzeug mit:
einer inneren Kabine;
einer Waschraumanordnung nach Anspruch 1, wobei der Luftströmungs-Generator dazu konfiguriert ist, eine Luftströmung entlang und/oder quer zu der Region der UV-Licht-Emission zu erzeugen; und wobei das UV-Licht-Desinfektionssystem ferner aufweist:
ein Gehäuse, wobei die UV-Licht-Anordnung innerhalb des Gehäuses festgelegt ist.

## Revendications

1. Bloc toilettes (200) formant un espace clos et comportant un système de désinfection par rayonnement ultraviolet (UV) qui est configuré pour désinfecter une surface d'une structure à l'intérieur du bloc toilettes, telle qu'une cuvette de WC (500), un plancher (502), des armoires (506) et/ou un lavabo (502), le système de désinfection par rayonnement UV comprenant :
un ensemble à rayonnement UV (104) comportant une source de rayonnement UV (110) qui est configurée pour émettre un rayonnement UV en direction de la surface de la structure, le rayonnement UV, grâce à ses photons de rayonnement UV, excitant des molécules d'oxygène d'un état fondamental à un état excité ; et
un générateur de flux d'air (106) qui est configuré pour générer un flux d'air à proximité de la source de rayonnement UV (110) et/ou de la surface à désinfecter et à l'intérieur d'une région d'émission de rayonnement UV entre la source de rayonnement UV (110) et la surface de la structure, le flux d'air généré circulant ensuite dans tout l'espace clos, en entraînant avec lui les molécules d'oxygène excitées, de manière à disperser les molécules d'oxygène excitées depuis la région proche de la source de rayonnement UV (110) jusque dans l'espace clos tout entier formé par le bloc toilettes de sorte que le flux d'air généré par le générateur de flux d'air (106) perturbe la formation d'ozone, le système de désinfection par rayonnement UV comprenant en outre une unité de commande de rayonnement UV (108) liée fonctionnellement à l'ensemble à rayonnement UV (104) et au générateur de flux d'air (106), l'unité de commande de rayonnement UV (108) étant configurée pour commander le fonctionnement de l'ensemble à rayonnement UV (104) et du générateur de flux d'air (106), l'unité de commande de rayonnement UV (108) étant configurée pour activer la source de rayonnement UV durant un cycle de désinfection, l'unité de commande de rayonnement UV (108) étant configurée pour activer le générateur de flux d'air (106) avant l'activation de la source de rayonnement UV durant le cycle de désinfection.

2. Bloc toilettes selon la revendication 1, le système de désinfection par rayonnement UV comprenant en outre un carter (102), l'ensemble à rayonnement UV (104) étant fixé à l'intérieur du carter (102).

3. Bloc toilettes selon la revendication 2, dans lequel le générateur de flux d'air (106) est fixé au carter (102).

4. Bloc toilettes selon l'une quelconque des revendications 1 à 3, dans lequel le générateur de flux d'air (106) comprend un ensemble ventilateur comportant au moins un ventilateur (118) lié fonctionnellement à au moins un actionneur (120).

5. Bloc toilettes selon l'une quelconque des revendications 1 à 4, dans lequel le générateur de flux d'air (106) est configuré pour générer un flux d'air le long de la région d'émission de rayonnement UV.

6. Bloc toilettes selon l'une quelconque des revendications 1 à 5, dans lequel le générateur de flux d'air (106) est configuré pour générer un flux d'air au travers de la région d'émission de rayonnement UV.

7. Procédé de désinfection par rayonnement ultraviolet (UV) pour désinfecter une surface d'une structure à l'intérieur d'un bloc toilettes (200), telle qu'une cuvette de WC (500), un plancher (502), des armoires (506) et/ou un lavabo (502), le procédé de désinfection par rayonnement UV comprenant :
l'émission d'un rayonnement UV en direction de la surface de la structure au moyen d'une source de rayonnement UV (110) d'un ensemble à rayonnement UV (104), le rayonnement UV convertissant des molécules d'oxygène en molécules d'ozone grâce à ses photons de rayonnement UV qui excitent des molécules d'oxygène d'un état fondamental à un état excité ;
l'utilisation d'un générateur de flux d'air (106) pour générer un flux d'air à proximité de la source de rayonnement UV (110) et/ou de la surface à désinfecter et à l'intérieur d'une région d'émission de rayonnement UV entre la source de rayonnement UV (110) et la surface de la structure, le flux d'air généré circulant ensuite dans tout l'espace clos, en entraînant avec lui les molécules d'oxygène excitées, de manière à disperser les molécules d'oxygène excitées depuis la région proche de la source de rayonnement UV (110) jusque dans l'espace clos tout entier formé par le bloc toilettes de sorte que le flux d'air généré par le générateur de flux d'air (106) perturbe la formation d'ozone ;
une unité de commande de rayonnement UV (108) étant liée fonctionnellement à l'ensemble à rayonnement UV (104) et au générateur de flux d'air (106) ;
la commande du fonctionnement de l'ensemble à rayonnement UV (104) et du générateur de flux d'air (106) au moyen de l'unité de commande de rayonnement UV (108) ;
l'activation de la source de rayonnement UV au moyen de l'unité de commande de rayonnement UV (108) durant un cycle de désinfection ; et
l'activation du générateur de flux d'air (106) au moyen de l'unité de commande de rayonnement UV (108) avant l'activation de la source de rayonnement UV durant le cycle de désinfection.

8. Procédé de désinfection par rayonnement UV selon la revendication 7, comprenant en outre la fixation de l'ensemble à rayonnement UV (104) à l'intérieur d'un carter (102).

9. Procédé de désinfection par rayonnement UV selon la revendication 7 ou 8, comprenant en outre la fixation du générateur de flux d'air (106) à un carter (102).

10. Procédé de désinfection par rayonnement UV selon l'une quelconque des revendications 7 à 9, comprenant en outre le positionnement du générateur de flux d'air (106) à distance d'un carter (102), le positionnement à distance comprenant la fixation du générateur de flux d'air (106) à moins de 12,7 cm (cinq pouces) d'une partie du composant.

11. Procédé de désinfection par rayonnement UV selon l'une quelconque des revendications 7 à 10, dans lequel l'utilisation du générateur de flux d'air comprend la génération d'un flux d'air le long de la région d'émission de rayonnement UV et/ou l'utilisation du générateur de flux d'air comprend la génération d'un flux d'air au travers de la région d'émission de rayonnement UV.

12. Véhicule, comprenant :
une cabine interne ;
un bloc toilettes selon la revendication 1, le générateur de flux d'air étant configuré pour générer un flux d'air le long et/ou au travers de la région d'émission de rayonnement UV ; et le système de désinfection par rayonnement UV comprenant en outre :
un carter, l'ensemble à rayonnement UV étant fixé à l'intérieur du carter.
